# EUROPÄISCHE PATENTSCHRIFT

(11) **EP 0 481 299 B1**
(45) Veröffentlichungstag und Bekanntmachung des Hinweises auf die Patenterteilung: **20.07.1994**
(21) Anmeldenummer: 91116915.9
(22) Anmeldetag: 04.10.1991
(51) Int. Cl.: C07D 211/58, C07D 401/12, A61K 31/445

(54) **N-Phenyl-piperidyl-4-amine und diese enthaltende Arzneimittel**
N-phenyl-piperidyl-4-amines and drugs containing them
N-phényl-pipéridyl-4-amines et médicaments les contenant

(30) Priorität: 16.10.1990 DE 4032766
(43) Veröffentlichungstag der Anmeldung: 22.04.1992
(73) Patentinhaber: BASF Aktiengesellschaft, 67063 Ludwigshafen (DE)
(72) Erfinder: Lubisch, Wilfried, Dr., W-6800 Mannheim 1 (DE); Schult, Sabine, Dr., W-6900 Heidelberg (DE); Binder, Rudolf, Dr., W-6520 Worms (DE); Raschack, Manfred, Dr., W-6714 Weisenheim am Sand (DE); Reinhardt, Roland, Dr., W-6750 Kaiserslautern (DE); Seemann, Dietmar, Dr., W-6907 Nussloch (DE)

(56) Entgegenhaltungen:
- EP-A- 0 369 627
- US-A- 4 198 411

## Beschreibung

Die Erfindung betrifft neue substituierte Phenylpiperidinoylamine, deren pharmazeutisch einsetzbare Salze und pharmazeutische Formulierungen, in denen diese als Wirkstoff enthalten sind.

Phenylpiperidinoylamine sind beschrieben in BE 678 063 (antiprotolytische Wirkung) und US 4,902,800 (Interleukin-I-Inhibitor). Ferner sind Phenylpiperidinoylamine mit antihistaminischer Wirkung beschrieben worden durch DRP 749887 (1941); E. Cerkovnikov et al., Chem. Ber. 74, 1648, 1658 und 1661 (1941) und V. Hahn et al., Helv. Chim. Akta 26, 1132 (1943).

Der Erfindung lag die Aufgabe zugrunde, neue Antiarrhythmika der Klasse III nach Vaughan Williams (vergleiche "Mechanisms and treatment of cardiac arrhythmias", Edit. H.J. Reiser und L.N. Horowitz, Verl. Urban und Schwarzenberg, Baltimore und München, 1985, Kapitel II.C) mit verbesserten Eigenschaften zu entwickeln.

Die Lösung dieser Aufgabe besteht in Phenylpiperidinoylaminen der Formel I 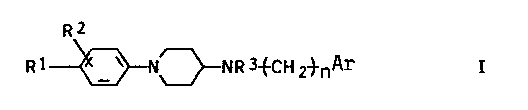
worin
- R¹: H, NO₂, R⁴SO₂NH, 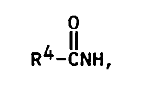 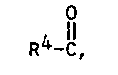 N≡C, CF₃, CF₃O, F, Cl, Br, C₁-C₄-Alkyl, R³O, CO₂R³, CHO, CH=NOR³, CH₃OR³ und
- R²: H, F, Cl, Br, C₁-C₄-Alkyl und R⁴O bedeuten, wobei R¹ und R² nicht gleichzeitig H sein können,
- R³: H und R⁴,
- R⁴: C₁-C₄-Alkyl und Phenyl,
- n: 1, 2, 3 und 4 und
- Ar: 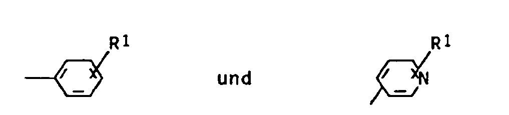 bedeutet, wobei R¹ unabhängig von R¹ in Formel I eine der dort angegebenen Bedeutungen hat,

sowie deren physiologisch verträglichen Salze.

Die erfindungsgemäßen Verbindungen können nach verschiedenen Analogie-Verfahren hergestellt werden:

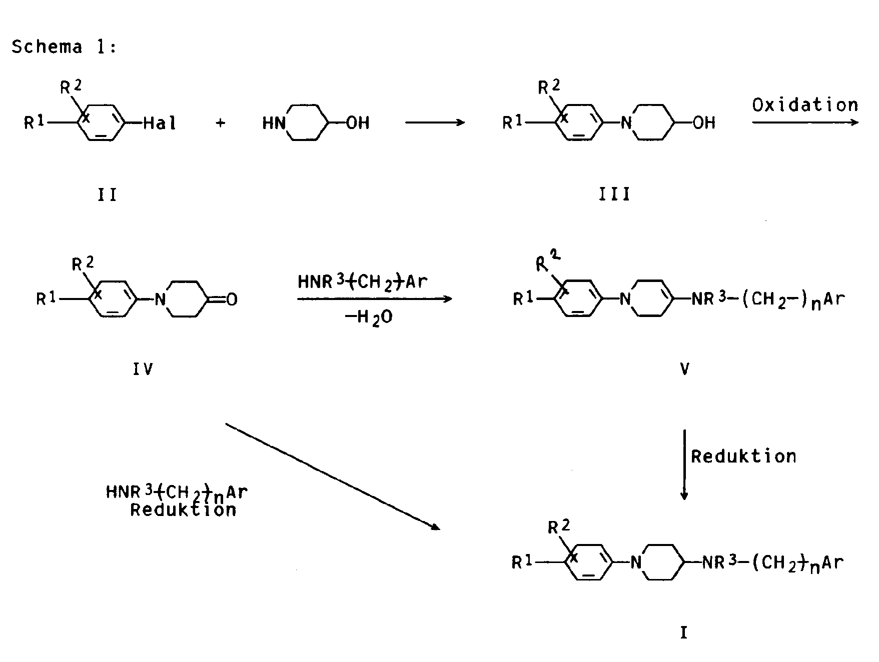

Der im Schema 1 aufgeführte Weg basiert auf einer von Taylor und Skotwicki beschriebenen Methode zur Darstellung von Derivaten der Verbindungen IV (Synthesis 1981, 606). Ausgehend vom aromatischen Halogenid II (Hal = F, Cl, Br), in dem R¹ eine elektronenziehende Gruppe darstellt, führt der Umsatz mit 4-Piperidinol in bevorzugt polaren Lösungsmitteln wie Dimethylformamid, Alkoholen und Ketonen bei erhöhter Temperatur, vornehmlich 60 bis 150°C, in Gegenwart von Basen, wie Kaliumkarbonat, zum Anilin-Derivat III. Ist R¹ keine elektronenziehende Gruppe, erfolgt die Reaktion bei erhöhter Temperatur, vornehmlich über 100°C unter Metall- bzw. Metallsalz-Katalyse, wobei insbesondere Kupfersalze oder Kupferpulver eingesetzt werden. Die Oxidation zu IV wird bevorzugt nach Pfitzner-Moffat (Dicyclohexylharnstoff/Dimethylsulfoxid) oder Swern (Oxalylchlorid oder Trifluoressigsäureanhydrid/Dimethylsulfoxid) in üblicher Weise ausgeführt. Das erfindungsgemäße Amin I erhält man durch reduktive Aminierung aus IV, wobei vornehmlich bei Raumtemperatur in Gegenwart von Reduktionsmitteln wie Natriumcyanoborhydrid oder Wasserstoff an z.B. Pd/Kohle, Pt/Kohle oder Raney-Nickel in polaren Lösungsmitteln wie Alkoholen gearbeitet wird. Alternativ kann aus IV und dem Amin HNR³(̵CH₂)̵ₙAr das Enamin V in üblicher Weise (aprotisches Lösungsmittel, bevorzugt Toluol; Säurekatalyse, bevorzugt p-Toluolsulfonsäure und Ameisensäure, erhöhte Temperatur) hergestellt werden, das danach in vornehmlich Alkoholen mit Reduktionsmitteln wie Natriumborhydrid oder Wasserstoff an den üblichen Metallkontakten wie Pd/Kohle oder Pt/Kohle zum Produkt I reduziert werden kann.

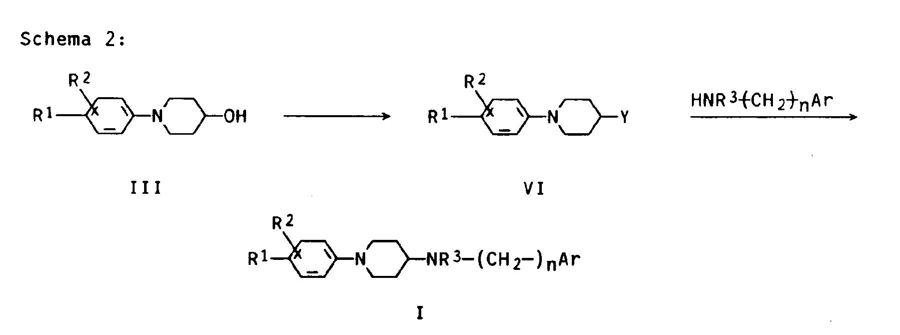

In Schema 2 wird der Alkohol III zu VI umgesetzt, wobei Y eine Abgangsgruppe wie Chlor oder Brom (die in üblicher Weise aus III durch Umsetzung mit Thionylchlorid oder Phosphortribromid eingeführt werden kann)
oder OSO₂CF₃ oder 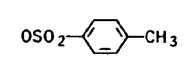
dargestellt (die durch Umsatz von III mit den entsprechenden Sulfonsäurechloriden bzw. -anhydriden eingeführt werden können). Die Reaktion von VI mit dem Amin HNR³(̵CH₂)̵ₙAr zu I erfolgt mit oder ohne Lösungsmittel bei Temperaturen von 25 bis 150°C, gegebenenfalls in Anwesenheit einer Base.

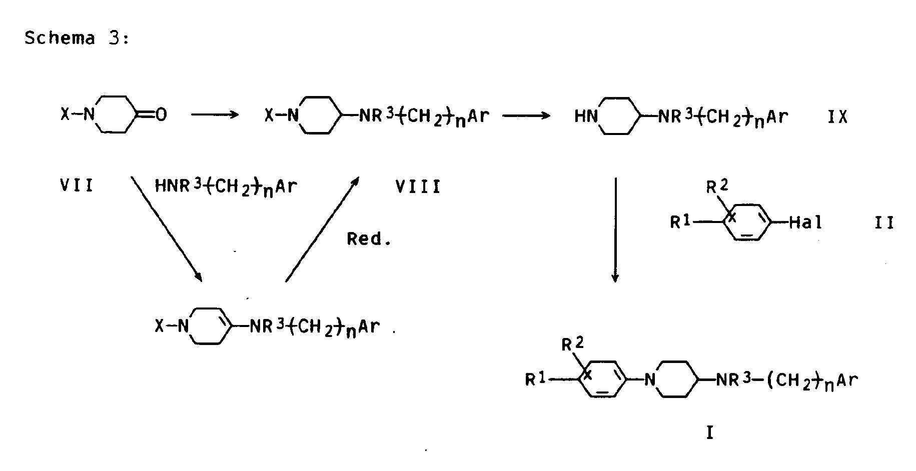

In der Variante 3 (Schema 3) erhält man das Produkt durch Umsatz des Amins IX mit dem Halogenid II in analoger Weise wie im Schema 1. Ausgehend von Piperidon VII, wobei X eine Schutzgruppe wie Benzyl, 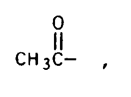 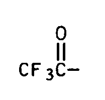
oder tert.-Butoxycarbonyl (BOC) darstellt, entsteht durch reduktive Aminierung mit dem Amin HNR³(̵CH₂)̵ₙAr VIII. Dabei wird in Lösungsmitteln wie Alkoholen und mit Reduktionsmitteln wie Natriumcyanoborhydrid oder Wasserstoff an Pd/Kohle, Pt/Kohle oder Raney-Nickel gearbeitet. Durch Abspaltung der Schutzgruppe X, entweder katalytisch mit Wasserstoff oder hydrolytisch mit HCl oder NaOH, entsteht das Amin IX. Alternativ kann aus VII und dem Amin HNR³(̵CH₂)̵ₙAr ein Enamin in üblicher Weise (aprotisches Lösungsmittel, bevorzugt Toluol; Säurekatalyse, bevorzugt p-Toluolsulfonsäure und Ameisensäure; erhöhte Temperatur) hergestellt werden, das danach in vornehmlich Alkoholen mit Reduktionsmitteln wie Natriumborhydrid oder Wasserstoff an den üblichen Metallkontakten wie Pd/Kohle oder Pt/Kohle zum Produkt VIII reduziert werden kann.

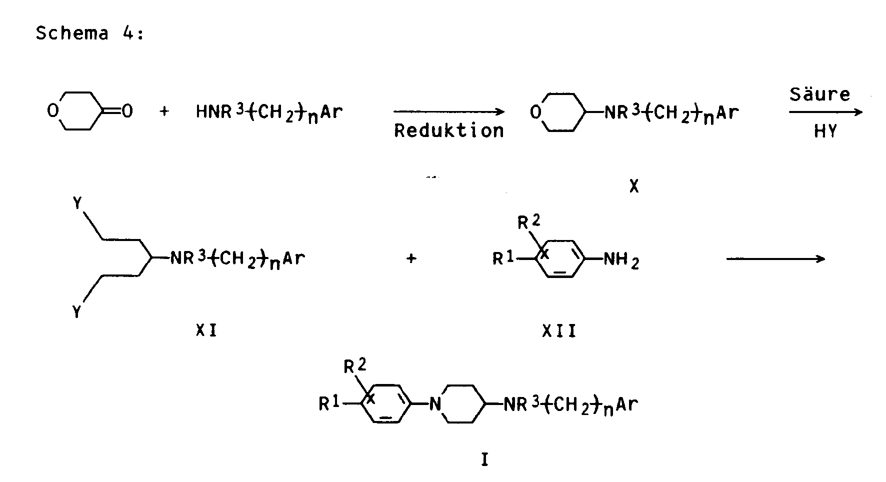

Ein weiterer Darstellungsweg geht vom Tetrahydro-4-pyranon aus (Schema 4), das in üblicher Weise, analog zur Darstellung von VIII in Schema 3, durch reduktive Aminierung zu X umgesetzt wird. X wird in einer konzentrierten Säure wie Bromwasserstoffsäure oder Salzsäure, gegebenenfalls in einem Lösungsmittel, bei erhöhter Temperatur in das Dihalogenid XI (Y = Halogen) überführt. Die Alkylierung des Anilins XII mit XI erfolgt in polaren Lösungsmitteln wie Alkoholen und Dimethylformamid oder ohne Lösungsmittel bei erhöhter Temperatur, wobei gegebenenfalls eine Base wie Kaliumcarbonat. anwesend ist.

Gegebenenfalls werden die so erhaltenen Phenylpiperidinoylamine in das Säureadditionssalz einer physiologisch verträglichen Säure übergeführt. Eine Zusammenstellung üblicher physiologisch verträglicher Salze kann aus Fortschritte der Arzneimittelforschung, 1966, Birkhäuser Verlag, Bd. 10, S. 224 bis 285, Deutschland, Schweiz, entnommen werden.

Die Säureadditionssalze werden in der Regel in an sich bekannter Weise durch Mischen der freien Base oder deren Lösungen mit der entsprechenden Säure oder deren Lösungen in einem organischen Lösungsmittel, beispielsweise einem niederen Alkohol, wie Methanol, Ethanol oder Propanol, oder einem niederen Keton, wie Aceton, Methylethylketon oder Methylisobutylketon, oder einem Ether wie Diethylether, Tetrahydrofuran oder Dioxan erhalten. Zur besseren Kristallabscheidung können Mischungen der genannten Lösungsmittel verwendet werden. Darüber hinaus können pharmazeutisch vertretbare wäßrige Lösungen von Säure-Additionsverbindungen der Phenylpiperidinoylamine der Formel I durch Auflösen der freien Base in einer wäßrigen Säurelösung hergestellt werden.

Die erfindungsgemäßen Phenylpiperidinoylamine stellen Antiarrhythmika der Klasse III dar. Zudem zeigen sie Affinität zum Sigma-Rezeptor und daher antipsychotische, antikonvulsive und neuroprotektive Wirkung. Weiterhin wurde gefunden, daß die Verbindungen den ATP-sensitiven K-Kanal blockieren.

Die Erfindung betrifft daher auch therapeutische Mittel zur topischen und vor allem systemischen Anwendung, die eine Verbindung der Formel I neben üblichen Trägerstoffen und/oder sonstigen galenischen Hilfsmitteln als Wirkstoff enthalten.

Die Herstellung der therapeutischen Mittel oder Zubereitungen erfolgt mit den üblichen flüssigen oder festen Trägerstoffen oder Verdünnungsmitteln und den üblicherweise verwendeten pharmazeutisch-technischen Hilfsstoffen, entsprechend der gewünschten Applikationsart und mit einer zur Anwendung geeigneten Dosierung, in üblicher Weise, beispielsweise durch Vermischen des Wirkstoffes mit den an sich in solchen Präparaten üblichen festen und flüssigen Träger- und Hilfsstoffen.

Die Mittel können peroral, parenteral oder topisch verabreicht werden. Derartige Zubereitungen sind beispielsweise Tabletten, Filmtabletten, Dragees, Kapseln, Pillen, Pulver, Lösungen oder Suspensionen, Infusions- oder Injektionslösungen sowie Pasten, Salben, Gele, Cremes, Lotionen, Puder, Lösungen oder Emulsionen und Sprays.

Die therapeutischen Mittel können die erfindungsgemäß zu verwendenden Verbindungen bei lokaler Anwendung in 0,01 bis 1 %iger Konzentration und bei systemischer Anwendung vorzugsweise in einer Einzeldosis von 0,1 bis 25 mg pro kg Körpergewicht enthalten und täglich in einer oder mehreren Dosierungen je nach Art und Schwere der Erkrankungen verabreicht werden.

Entsprechende Arzneimittel enthalten daher zweckmäßig 50 bis 1750 mg Wirkstoff pro Einzeldosis.

Üblicherweise verwendete pharmazeutisch-technische Hilfsstoffe sind beispielsweise für die lokale Anwendung Alkohole, wie Ethanol, Isopropanol, oxethyliertes Ricinusöl oder oxethyliertes hydriertes Ricinusöl, Polyacrylsäure, Glycerinmonostearat, Paraffinöl, Vaseline, Wollfett, Polyethylenglykol, Polypropylenglykol, Stearat sowie ethoxylierter Fettalkohol, für die systemische Anwendung Milchzucker, Propylenglykol und Ethanol, Stärke, Talk, Polyvinylpyrrolidon. Den Präparaten können gegebenenfalls ein Antioxidationsmittel, beispielsweise Tocopherol sowie butyliertes Hydroxyanisol oder butyliertes Hydroxytoluol, oder geschmacksverbessernde Zusätze, Stabilisierungsmittel, Emulgiermittel, Bleichmittel usw. zugesetzt werden. Voraussetzung ist, daß alle bei der Herstellung pharmazeutischer Zubereitungen verwendeten Stoffe toxikologisch unbedenklich und mit den verwendeten Wirkstoffen verträglich sind.

### Herstellung von Ausgangs- und Zwischenprodukten:

### Präparation 1

1,0 g (4,5 mmol) 1-(4-Nitrophenyl)-4-hydroxypiperidin werden in üblicher Weise mit Pd/Kohle in Methanol hydriert. Man erhält 0,8 g 1-(4-Aminophenyl)-4-hydroxypiperidin. Schmp. 176°C

### Präparation 2

20 g (0,10 mol) des Produktes aus Präparation 1 und 10,5 g (0,10 mol) Triethylamin werden in Methylenchlorid gelöst und bei 0°C tropfenweise mit 9,0 g (0,11 mol) Acetylchlorid, in Methylenchlorid gelöst, versetzt. Man rührt alles 3 h bei 0°C und gießt das Reaktionsgemisch anschließend in Wasser. Die wäßrige Phase wird mit Natriumchlorid gesättigt, wobei das Produkt ausfällt. Man erhält 18,6 g 1-(4-Acetaminophenyl)-4-hydroxypiperidin. Schmp. 191°C

### Präparation 3

18,0 g (76,8 mmol) des Produktes aus der Präparation 2 werden in 300 ml Dimethylsulfoxid/Toluol (= 1:2) gelöst und nacheinander mit 6,2 ml (76,8 mmol) Pyridin, 50,0 g (240 mmol) Dicyclohexylcarbodiimid und bei 0°C tropfenweise mit 3,0 ml (38,4 mmol) Trifluoressigsäure versetzt. Man rührt alles 16 h bei Raumtemperatur, gießt anschließend das Reaktionsgemisch in Wasser und extrahiert mit Essigsäureethylester. Die organische Phase wird getrocknet und i. Vak. eingeengt. Der Rückstand wird chromatographisch gereinigt (Fließmittel: Toluol/Aceton = 1:1). Man erhält 13,2 g 1-(4-Acetaminophenyl)-4-piperidon. Schmp. 158-159°C

### Präparation 4

Das Produkt aus der Präparation 1 wird analog dem Beispiel 9 mit Methansulfonylchlorid in Tetrahydrofuran umgesetzt. Man erhält das 1-(4-Methansulfonylaminophenyl)-4-hydroxypiperidin. Schmp. 126-129°C.

### Präparation 5

Das Produkt aus Präparation 4 wird analog zur Präparation 3 mit Dicyclohexylcarbodiimid/DMSO umgesetzt. Man erhält das 1-(4-Methansulfonylaminophenyl)-4-piperidon.

### Präparation 6

Zu 7,1 g (50 mmol) N-Acetylpiperidin und 6,1 g (50 mmol) N,N-Benzylmethylamin wurden bei 25°C 17,8 g (63 mmol) Titantetraisopropylat zugetropft. Nach einstündigem Rühren wurde die entstandene zähe Masse in 50 ml Ethanol aufgenommen und anschließend portionsweise mit 2,1 g (30 mmol) Natriumcyanoborhydrid versetzt. Man ließ alles bei Raumtemperatur 20 Stunden rühren. Danach tropfte man 10 ml Wasser zu, filtrierte den anorganischen Niederschlag ab und engte das Filtrat im Vakuum ein. Der anfallende Rückstand wurde zwischen Essigester und Wasser verteilt, die organische Phase abgetrennt, getrocknet und erneut im Vakuum eingeengt. Man erhielt 10,0 g des 1-Acetyl-4-(N,N-benzylmethylamino)-piperidins, Schmp. 44-45°C.

### Präparation 7

9,5 g (38,4 mmol) des Produktes aus Präparation 6 wurden in 200 ml Ethanol/4 M Natronlauge (1:1) gelöst und 10 Stunden unter Rückfluß gekocht. Anschließend wurde das Ethanol im Vakuum entfernt, der Rückstand mit Wasser verdünnt und alles mit Essigester extrahiert. Die organische Phase wurde getrocknet und im Vakuum rotierend eingedampft. Man erhielt 6,5 g des 4-(N,N-Benzylmethylamino)-piperidins als Öl.

¹H-NMR (CDCl₃): δ = 1.4-1.6 (2H), 1.7-1.9 (3H), 2.1 (3H), 2.5-2.7 (3H), 3.05 (2H), 3.55 (2H) und 7.15-7.3 (5H) ppm.

### Präparation 8

28,4 g (0,15 Mol) N-Benzyl-4-piperidon, 20,3 g (0,15 Mol) N,N-Methyl-(2-phenyl-1-ethyl)amin und 5 ml Ameisensäure wurden in 250 ml Toluol in einem Wasserabscheider bis zum Ende der Wasserentwicklung unter Rückfluß gekocht. Das Gemisch wurde anschließend im Vakuum einrotiert und das so erhaltene 1-Benzyl-4-(N,N-methyl-(2-phenyl-1-ethyl)-amino)-1,2,5,6-tetrahydropyridin als Rohprodukt direkt weiter umgesetzt.

Dazu wurde es in 500 ml Ethanol gelöst und bei 10°C portionsweise mit 17,0 g (0,45 Mol) Natriumborhydrid versetzt. Nach 16-stündigem Rühren bei Raumtemperatur wurde alles im Vakuum einrotiert und der Rückstand zwischen Methylenchlorid und Wasser verteilt. Die organische Phase wurde getrocknet und im Vakuum einrotiert. Das so erhaltene Öl wurde als Dioxalat kristallisiert. Man erhielt 57,4 g des 1-Benzyl-4-(N,N-methyl-(2-phenylethyl)-amino)-piperidin-dioxalats. Schmp. 203-204°C (i-Propanol).

### Präparation 9

32,8 (0,1 Mol) des Produktes aus Präparation 8 wurden in 500 ml Methanol gelöst und nach Zugabe von 5 g Palladium/Kohle (10 %) hydriert. Anschließend wurde filtriert und das Filtrat im Vakuum einrotiert. Das anfallende Öl kristallisierte als Dioxalat. Man erhielt 35,8 g 4-(N,N-Methyl-(2-phenylethyl)-amino)piperidindioxalat. Schmp. 169-170°C.

### Präparation 10

17,1 g (0,14 Mol) N-Acetyl-4-piperidon und 16,9 g (0,14 mol) N,N-(4-Fluorbenzyl)-methylamin wurden analog Präparation 6 umgesetzt. Man erhielt 25,1 g des 1-Acetyl-4-(N,N-(4-fluorbenzyl)methylamino)piperidins.

¹H-NMR (CDCl₃): δ = 1.4-1.6 (2H), 1.75-1.9 (2H), 2.1 (3H), 2.2 (3H), 2.4-2.7 (2H), 3.0 81H), 3.5 (2H), 3.9 (1H), 4.7 (1H), 7.0 (2H) und 7.25 (2H) ppm.

### Präparation 11

26,5 (0,10 Mol) des Produktes aus Präparation 10 wurden analog Präparation 7 hydrolysiert. Man erhielt 19,7 g des 4-(N,N-(4-Fluorbenzyl)methylamino)piperidins als Öl.

¹H-NMR (CDCl₃): δ = 1.4-1.6 (2H), 1.8-1.9 (2H), 2.1 (3H), 2.4-2.7 (4H), 3.2 (2H), 6.9-7.05 (2H) und 7.2-7.3 (2H) ppm.

### Präparation 12

1,8 g (4,7 mmol) des Produktes aus Beispiel 40 wurden in 100 ml Methanol gelöst und in Gegenwart von 0,3 g Palladium/Aktivkohle (10 %ig) hydriert. Anschließend wurde filtriert und das Filtrat im Vakuum einrotiert. Man erhielt 1,3 g 1-(4-Aminophenyl)-4-(N,N-(2-(4-aminophenyl)-ethyl)amino)piperidin als Öl.

¹H-NMR (D₆-DMSO): δ = 1.4-1.6 (2H), 1.7-1.8 (2H), 2.1 (3H), 2.3-2.7 (7H), 3.4 (2H), 4.0-4.6 (4H), 6.4 (4H), 6.7 (2H) und 6.85 (2H) ppm.

### Präparation 13

1,5 g (4,0 mmol) des Amins aus Beispiel 36 wurden in 100 ml Ethanol suspendiert und mit einer Lösung von 0,26 g Kupfer-II-sulfatpentahydrat in 0,5 ml Wasser versetzt. Anschließend gab man 2 g Natriumborhydrid portionsweise zu und erwärmte alles vorsichtig 2 Stunden auf Rückfluß. Der Ansatz wurde auf Wasser gegossen und mit Essigester extrahiert. Die organische Phase wurde getrocknet und im Vakuum einrotiert. Man erhielt 0,79 g des 1-(4-Aminophenyl)-4-(N,N-(4-amino-benzyl)methylamino)piperidins. Schmp. 123°C.

### Präparation 14

1,5 g (4,4 mmol) des Amins aus Beispiel 33 wurde in 100 ml Methanol gelöst und in Gegenwart von 0,5 g Platin/Aktivkohle (5 %ig) hydriert. Danach wurde filtriert und das Filtrat im Vakuum einrotiert. Man erhielt 1,1 g des 1-(4-Aminophenyl)-4-(N,N-(4-fluorbenzyl)methylamino)piperidins als Öl.

¹H-NMR (D₆-DMSO): δ = 1.5-1.7 (2H), 1.8 (2H), 2.1 (3H), 2.35-2.6 (3H), 3.4 (2H), 3.55 (2H), 4.5-4.7 (2H), 6.5 (2H), 6.7 (2H), 7.1 (2H) und 7.35 (2H) ppm.

### Beispiele

### Beispiel 1

3,3 g (16,2 mmol) 1-(4-Cyanphenyl)-4-piperidon, 1,0 g (16,2 mmol) Essigsäure und 4,0 g (32,4 mmol) Benzylmethylamin wurden in 50 ml Methanol gelöst und portionsweise mit 1,0 g (16,2 mmol) Natriumcyanborhydrid versetzt. Man rührte alles 2 h bei Raumtemperatur. Anschließend wurde das Reaktionsgemisch i. Vak. eingeengt und der Rückstand zwischen Wasser und Essigsäureethylester verteilt. Die organische Phase wurde chromatographisch gereinigt (Fließmittel: Toluol/Aceton = 2:1) und in üblicher Weise mit etherischer Chlorwasserstoff-Lösung versetzt. Man erhielt 2,8 g 1-(4-Cyanphenyl)-4-(N-benzyl-N-methylamino)-piperidindihydrochlorid. Schmp. 211-212°C

### Beispiel 2

Zu 5,0 g (25 mmol) 1-(4-Cyanphenyl)-4-piperidon und 4,5 g (25 mmol) N-Methyl-2-(4-nitrophenyl)-ethylamin in 150 ml Methanol wurden nacheinander 1,5 g (25 mmol) Essigsäure und 1,6 g (25 mmol) Natriumcyanborhydrid gegeben. Man rührte 1 h bei Raumtemperatur und entfernte anschließend das Lösungsmittel i. Vak. Der Rückstand wurde zwischen verdünnter Natronlauge und Essigsäureethylester verteilt, die organische Phase abgetrennt, getrocknet und i. Vak. eingeengt. Das erhaltene Öl wurde in üblicher Weise mit etherischer Chlorwasserstoff-Lösung behandelt. Man erhielt das 1-(4-Cyanphenyl)-4-(N-methyl-2-(4-nitrophenyl)-ethylamino)-piperidindihydrochlorid. Schmp. 174-178°C

### Beispiel 3

1,2 g (3,3 mmol) des Produktes aus Beispiel 2 wurden in 100 ml Tetrahydrofuran in üblicher Weise an Pd/C (10 %ig) hydriert und aufgearbeitet. Man erhielt 1,1 g des 4-(2-(4-Aminophenyl)-ethyl-N-methylamino)-1-(4-cyanphenyl)-piperidins als Öl. ¹H-NMR (d₆-DMSO) δ = 1,2-1,5 (2H); 1,8-1,9 (2H); 2,1 (3H); 2,4-2,7 (4H); 2,7-2,9 (2H); 3,3 (1H); 3,9 (2H); 6,4 (2H); 6,8 (2H); 6,9 (2H) und 7,5 (2H) ppm.

### Beispiel 4

Zu 1,0 g (3 mmol) des Produktes aus Beispiel 3 und 0,6 g (6 mmol) Triethylamin in 100 ml wasserfreiem Tetrahydrofuran wurden bei 0°C 0,34 g (3 mmol) Methansulfonylchlorid, gelöst in Tetrahydrofuran, zugetropft. Man rührte 16 h bei Raumtemperatur und entfernte anschließend das Lösungsmittel im Vakuum. Der Rückstand wurde zwischen wäßriger Natriumhydrogenkarbonatlösung und Methylenchlorid verteilt, die organische Phase getrocknet und im Vakuum eingeengt. Das erhaltene Öl wurde chromatographisch gereinigt (Fließmittel: Methylenchlorid/Methanol = 10:1). Man erhielt 0,73 g des 1-(4-Cyanphenyl)-4-(2-(4-methansulfonyl-aminophenyl)-ethyl-N-methylamino)-piperidins. ¹H-NMR (d₆-DMSO) δ = 1,3-1,5 (2H); 1,7 (2H); 2,1 (3H); 2,5-2,7 (4H); 2,7-3,0 (5H); 3,3 (1H); 3,9 (2H); 6,9 (2H); 7,0-7,2 (4H); 7,5 (2H) und 9,6 (1H) ppm.

### Beispiel 5

2,5 g (11,4 mmol) 1-(4-Nitrophenyl)-4-piperidon, 1,5 g (11,4 mmol) N-Methyl-N-(2-phenylethyl)-amin, 0,7 g (11,4 mmol) Essigsäure und 0,7 g (11,4 mmol) Natriumcyanoborhydrid wurden analog zu Beispiel 1 umgesetzt. Das Produkt wurde in üblicher Weise mit Fumarsäure zum Fumarat umgesetzt. Man erhielt 2,8 g 4-(N-Methyl-N-(2-phenylethyl)-amino)-1-(4-nitrophenyl)-piperidin-fumarat. Schmp. 201-202°C

### Beispiel 6

1-(4-Nitrophenyl)-4-piperidon wurde analog Beispiel 2 mit N-Benzyl-N-methylamin und anschließend mit Fumarsäure umgesetzt. Man erhielt 4-(N-Benzyl-N-methylamino)-1-(4-nitrophenyl)-piperidinfumarat. Schmp. 183°C

### Beispiel 7

Das Produkt aus Präparation 3 wurde analog Beispiel 5 mit N-Benzyl-N-methylamin umgesetzt. Man erhielt 1-(4-Acetaminophenyl)-4-(N-benzyl-N-methylamino)-piperidin. Schmp. 147°C

### Beispiel 8

5,5 g (16,3 mmol) des Produktes aus Beispiel 7 wurden in einem Gemisch aus 100 ml 2 M Natronlauge und 200 ml Methanol 12 h am Rückfluß gekocht. Das Reaktionsgemisch wurde anschließend i. Vak. eingeengt, der Rückstand zwischen Wasser und Methylenchlorid verteilt, die organische Phase getrocknet und i. Vak. eingeengt. Man erhielt 2,8 g 1-(4-Aminophenyl)-4-(N-benzyl-N-methylamino)-piperidin als Öl, das als Difumarat kristallisierte. Schmp. 138°C

### Beispiel 9

Das Produkt aus Beispiel 8 wurde analog Beispiel 4 mit Methansulfonylchlorid umgesetzt und anschließend ins Hydrochlorid überführt. Man erhielt das 4-(N-Benzyl-N-methylamino)-1-(4-methansulfonylaminophenyl)-piperidindihydrochlorid. Schmp. 221°C (Zers.)

### Beispiel 10

Das Produkt aus der Präparation 5 wurde analog Beispiel 5 mit N-Methyl-N-(2-(4-nitrophenyl)-ethyl)-amin umgesetzt. Man erhielt 1-(4-Methansulfonylaminophenyl)-4-[N-methyl-N-(2-(4-nitrophenyl)-ethyl)-amino]-piperidin. Schmp. 225-226°C

### Beispiel 11

Das Produkt aus der Präparation 5 wurde analog Beispiel 5 mit N-Methyl-4-picolylamin umgesetzt. Man erhielt 1-(4-Methan-sulfonyl-aminophenyl)-4-(N-methyl-4-picolylamino)-piperidin als Öl. ¹H-NMR (d₆-DMSO) δ = 1,4-1,7 (2H); 1,7-1,9 (2H); 2,1 (3H); 2,4-2,7 (4H); 3,5-3,9 (3H); 6,9 (2H); 7,0 (2H); 7,3 (2H); 8,5 (2H); und 9,2 (1H) ppm.

### Beispiel 12

18,8 g (86 mmol) des in Präparation 9 dargestellten Amins, 11,9 g (86 mmol) des 4-Fluoracetophenons und 47,5 g (344 mmol) Kaliumcarbonat wurden in 250 ml Diemthylformamid 20 h unter Rückfluß gekocht. Anschließend wurde das Gemisch mit viel Wasser verdünnt und mit Essigester extrahiert. Die organische Phase wurde getrocknet und im Vakuum einrotiert. Der Rückstand wurde als Fumarat kristallisiert. Man erhielt 18,1 g des 1-(4-Acetylphenyl)-4-(N,N-methyl-(2-phenylethyl)amino) piperidin-fumarats. Schmp. 155-157°C.

### Beispiel 13

Zu 10,4 g (0,32 mol) des Produktes aus Beispiel 12 (als Base), das in 500 ml Ethanol gelöst wurde, wurden bei 10°C 18,0 g (0,475 mol) Natriumborhydrid portionsweise zugefügt. Nach 16-stündigem Rühren bei Raumtemperatur wurde alles im Vakuum einrotiert. Der Rückstand wurde zwischen Methylenchlorid und Wasser verteilt, die organische Phase getrocknet und im Vakuum einrotiert. Das so erhaltene Öl wurde als Fumarat kristallisiert. Man erhielt 16,4 g des 1-(4-(1-Hydroxy-1-ethyl)phenyl)-4-(N,N-methyl-(2-phenyl-1-ethyl)amino)piperidindifumarats. Schmp. 124-129°C.

### Beispiel 14

Ein Gemisch aus 5,1 g (15 mmol) des Produktes aus Beispiel 13, 2,6 g (22,5 mmol) Triethylsilan und 17,1 g (15 mmol) Trifluoressigsäure wurde 2 Stunden auf 60°C erwärmt. Danach wurde alles auf Wasser gegossen, mit 4 M Natronlauge alkalisch gestellt und mit Methylenchlorid extrahiert. Die organische Phase wurde abgetrennt, getrocknet und im Vakuum einrotiert. Der erhaltene Rückstand wurde chromatographisch an Kieselgel (Fließmittel: Toluol/Aceton = 2/1) gereinigt. Das ölige Produkt kristallisierte als Fumarat. Man erhielt 1,5 g des 1-(4-Ethylphenyl)-4-(N,N-methyl(2-phenyl-1-ethyl)amino)piperidinfumarats. Schmp. 163-165°C.

### Beispiel 15

4,0 g (20 mmol) des Produktes aus Präparation 7, 3,1 g (20 mmol) 4-Fluorbenzoesäuremethylester und 5,5 g (40 mmol) Kaliumcarbonat wurden analog Beispiel 1 umgesetzt. Man erhielt 2,1 g des 4-(N,N-Benzyl-methylamino)-1-(4-methoxycarbonylphenyl)-piperidins. Schmp. 103-104°C.

### Beispiel 16

4,5 g (13,2 mmol) des Produktes aus Beispiel 15 und 0,55 g Natriumhydroxid wurden in 150 ml Ethanol/Wasser (1:1) gelöst und 6 Stunden unter Rückfluß gekocht. Das Ethanol wurde anschließend im Vakuum entfernt und die wäßrige Phase mit 1 M Salzsäure neutralisiert, wobei das Produkt ausfiel. Man erhielt 3,3 g des 4-(N,N-Benzylmethylamino)-1-(4-carboxyphenyl)piperidins. Schmp. 227-228°C.

### Beispiel 17

8,2 g (40 mmol) des Produktes aus Präparation 7, 5,5 g (40 mmol) des 4-Fluoracetophenons und 11,0 g (40 mmol) Kaliumcarbonat wurden analog Beispiel 12 umgesetzt. Man erhielt 5,5 g des 1-(4-Acetylphenyl)-4-(N,N-benzylmethylamino)-piperidinfumarats. Schmp. 163-164°C.

### Beispiel 18

2,5 g (7,9 mmol) des Produktes aus Beispiel 17 wurden in 50 ml Eisessig gelöst und bei Raumtemperatur mit 1,26 g (7,9 mmol) Brom, das in 10 ml Eisessig gelöst war, tropfenweise versetzt. Man ließ alles 4 Stunden rühren. Danach wurde das Gemisch mit Ether versetzt, wobei sich langsam Kristalle abschieden. Das Kristallisat wurde abfiltriert und mit Ether gewaschen. Man erhielt 3,5 g 1-(4-Acetyl-2-bromphenyl)-4-(N,N-benzylmethylamino)piperidinhydrobromid. Schmp. 201-202°C.

### Beispiel 19

6,7 g (30 mmol) des Produktes aus Präparation 11, 4,1 g (30 mmol) 4-Fluoracetophenon und 8,3 g (60 mmol) Kaliumcarbonat wurden analog Beispiel 12 umgesetzt. Man erhielt 4,6 g 1-(4-Acetylphenyl)-4-(N,N-(4-fluorbenzyl)methylamino)-piperidin. Schmp. 133°C.

### Beispiel 20

2,4 g (7 mmol) des Produktes aus Beispiel 19 wurden analog Beispiel 14 mit 2,4 g (20,9 mmol) Triethylsilan und 7,9 g (70 mmol) Eisessig reduziert. Man erhielt 2,3 g des 1-(4-Ethylphenyl)-4-(N,N-(4-fluorbenzyl)-methylamino)piperidinfumarats. Schmp. 150-152°C.

### Beispiel 21

8,2 g (40 mmol) des Produktes aus Präparation 7, 5 g (40 mmol) 4-Fluorbenzaldehyd und 11,1 g (80 mmol) Kaliumcarbonat wurden analog Beispiel 12 umgesetzt. Man erhielt 8,4 g des 4-(N,N-Benzylmethylamino)-1-(4-formylphenyl)piperidinfumarats. Schmp. 129-133°C.

### Beispiel 22

1,5 g (5 mmol) des Produktes aus Beispiel 21 und 0,7 g (10 mmol) Hydroxylaminhydrochlorid wurden in 30 ml Ethanol 5 Stunden unter Rückfluß gekocht. Beim Abkühlen kristalliserten 1,2 g des 4-(N,N-Benzylmethylamino)-1-(4-(hydroxyliminomethyl)-phenyl)-piperinhydrochlorids. Schmp. 238-240°C.

### Beispiel 23

7,5 g (24 mmol) des Produktes aus Beispiel 21 wurden analog Beispiel 13 mit 1,0 g Natriumborhydrid reduziert. Man erhielt 4,1 g des 4-(N,N-Benzylmethylamino)-1-(4-(hydroxymethyl)phenyl)piperidintartrats als amorphen Festkörper.

¹H-NMR (D₆-DMSO): δ = 1.6-1.8 (2H), 1.9-2.0 (2H), 2.15 (3H), 2.5-2.7 (2H), 2.8 (1H), 2.7-2.9 (4H), 4.25 (2H; Tartrat), 4.35 (2H), 6.0 (breit), 6.9 (2H), 7.2 (2H) und 7.3-7.5 (5H) ppm.

### Beispiel 24

1,9 g (6,1 mmol) der Base des Produkts aus Beispiel 23 wurden analog Beispiel 14 mit 1,1 g (9,2 mmol) Triethylsilan und 8,0 g Eisessig reduziert. Man erhielt 2,0 g des 4-(N,N-Benzylmethylamino)-1-(4-tolyl)piperidinfumarats. Schmp. 161-163°C.

### Beispiel 25

6,7 g (30 mmol) des Amins aus Präparation 11, 3,7 g (30 mmol) 4-Fluorbenzaldehyd und 8,3 g (60 mmol) Kaliumcarbonat wurden analog Beispiel 12 umgesetzt. Man erhielt 10,8 g des 4-(N,N-(4-Fluorbenzyl)methylamino)-1-(4-formylphenyl)piperidinfumarats. Schmp. 114-116°C.

### Beispiel 26

2,6 g (7,9 mmol) des Amins von Beispiel 25 wurden analog Beispiel 14 mit 2,8 g (23,7 mmol) Triethylsilan und 9,0 g Trifluoressigsäure reduziert. Man erhielt 1,7 g des 4-(N,N-(4-Fluorbenzyl)methylamino)-1-(4-tolyl)-piperidinfumarats. Schmp. 146°C.

### Beispiel 27

9,3 g (60 mmol) 4-Fluorbenzoesäuremethylester, 13,3 g (60 mmol) des Amins aus Präparation 11 und 16,6 g (120 mmol) Kaliumcarbonat wurden analog Beispiel 12 umgesetzt. Man erhielt 8,4 g des 4-(N,N-(4-Fluorbenzyl)methylamino)-1-(4-methoxycarbonylphenyl)piperidins. Schmp. 92°C.

### Beispiel 28

2,5 g (7 mmol) des Produktes aus Beispiel 27 wurden in 140 ml 1 M Natronlauge/Ethanol (1:1) 5 Stunden unter Rückfluß gekocht. Anschließend wurde analog Beispiel 16 aufgearbeitet. Man erhielt 1,8 g des 1-(4-Carboxyphenyl)-4-(N,N-(4-fluorbenzyl)methylamino)piperidins. Schmp. 256-258°C.

### Beispiel 29

1,8 g (5,3 mmol) des Produktes aus Beispiel 28 wurden in 50 ml Methylenchlorid gelöst, mit 2,5 g (21,3 mmol) Thionylchlorid versetzt und 5 Stunden unter Rückfluß gekocht. Die so erhaltene Lösung wurde zu einer bei 0°C gut gerührten Mischung aus 100 ml wäßriger Ammoniak-Lösung und 100 ml Methylenchlorid zugetropft. Anschließend wurde die organische Phase abgetrennt, getrocknet und im Vakuum einrotiert. Der Rückstand wurde chromatographisch gereinigt und als Tartrat kristallisiert. Man erhielt 0,4 g des 1-(4-Carbamoylphenyl)-4-(N,N-(fluorbenzyl)methylamino)piperidintartrats. Schmp. 204°C.

### Beispiel 30

Lösung A: 2,6 g Kupfer-II-sulfatpentahydrat und 0,9 g Natriumchlorid wurden in 8 ml Wasser gelöst und mit einer weiteren Lösung, die aus 0,65 g Natriumsulfit und 2 ml Wasser hergestellt wurde, versetzt. Ein ausgefallener Niederschlag wurde mit 4,1 ml konzentrierter Salzsäure gelöst und zu der obigen Lösung zugefügt.

2,3 g (7,7 mmol) des Amins aus Beispiel 8 wurden in 4,6 ml halbkonzentrierter Salzsäure gelöst. Zu der auf unter 5°C gekühlten Lösung tropfte man 3,1 ml einer 2,5 M Natriumnitrit-Lösung. Die so erhaltene Lösung der Diazoverbindung wurde anschließend bei 0°C in die Lösung A eingetragen und alles bis zum Ende der Stickstoffentwicklung erwärmt. Der Ansatz wurde danach in 2 M Natronlauge gegossen. Diese wäßrige Phase wurde mit Methylenchlorid extrahiert. Die organische Phase wurde abgetrennt, getrocknet und im Vakuum einrotiert. Das anfallende Öl wurde als Fumarat kristallisiert. Man erhielt 1,1 g des 4-(N,N-Benzylmethylamino)-1-(4-chlorphenyl)piperidinfumarats. Schmp. 151-153°C.

### Beispiel 31

3,1 g (15 mmol) des Amins aus Präparation 7, 2,5 g (15 mmol) 4-Fluorbenzotrifluorid und 4,2 g (30 mmol) Kaliumcarbonat wurden analog Beispiel 12 umgesetzt. Man erhielt 1,4 g des 4-(N,N-Benzylmethylamino)-1-(4-(trifluormethyl)phenyl)piperidinfumarats. Schmp. 162-164°C.

### Beispiel 32

3,3 g (15 mmol) des Amins aus Präparation 11, 1,8 g (15 mmol) 4-Fluorbenzonitril und 4,2 g (30 mmol) Kaliumcarbonat wurden analog Beispiel 12 umgesetzt. Man erhielt 2,95 g des 1-(4-Cyanophenyl)-4-(N,N-(4-fluorbenzyl)methylamino)piperidins. Schmp. 100-101°C.

### Beispiel 33

12,0 g (54 mmol) des Amins aus Präparation 11, 7,6 g (54 mmol) 4-Fluor-1-nitrobenzol und 7,5 g (54 mmol) Kaliumcarbonat wurden analog Beispiel 12 umgesetzt. Man erhielt 15,4 g des 4-(N,N-(4-Fluorbenzyl)methylamino)-1-(4-nitrophenyl)piperidins. Schmp. 89-90°C.

### Beispiel 34

Zu 3,2 g (12,0 mmol) des Produktes aus Präparation 5 und 2,0 g (12,0 mmol) N,N-Methyl-(p-nitrobenzyl)amin tropfte man 4,3 g (15,0 mmol) Titan-(IV)-isopropylat. Nach 1-stündigem Rühren bei Raumtemperatur gab man 25 ml wasserfreies Tetrahydrofuran zu. Danach wurden 0,5 g (8,0 mmol) Natriumcyanoborhydrid portionsweise zugefügt und 3 Stunden gerührt. Das Reaktionsgemisch wurde mit 2,4 ml Wasser hydrolysiert, der entstandene Niederschlag abgesaugt und das Filtrat mit Methylenchlorid extrahiert. Die organische Phase wurde abgetrennt, getrocknet und im Vakuum einrotiert. Man erhielt 1,8 g des 1-(4-Methansulfonamidophenyl)-4-(N,N-methyl-(4-nitrobenzyl)amino)piperidins. Schmp. 167-168°C.

### Beispiel 35

4,0 g (12,3 mmol) des Produktes aus Präparation 12 und 1,92 ml (24,7 mmol) Methansulfonsäurechlorid wurden analog Beispiel 4 umgesetzt. Man erhielt 1,2 g des 1-(4-Methansulfonamidophenyl)-4-(N,N-2(4-methansulfonamidphenyl)-1-ethyl)-methylamino)piperidins.

¹H-NMR (D₆-DMSO): δ = 1.3-1.5 (2H), 1.7-1.8 (2H), 2.3 (3H), 2.4-2.7 (7H), 2.85 (3H), 2.9 (3H), 3.6 (2H), 6.9 (2H), 7.0-7.3 (6H), 9.2 (1H) und 9.5 (1H).

### Beispiel 36

2,7 g (12,3 mmol) 1-(4-Nitrophenyl)-4-piperidon und 2,0 g (12,3 mmol) N,N-Methyl(4-nitrobenzyl)amin wurden analog Beispiel 5 umgesetzt. Man erhielt 3,0 g des 4-(N,N-Methyl-(4-nitrobenzyl)amino)-1-(4-nitrophenyl)piperidins. Schmp. 119-120°C.

### Beispiel 37

4,0 g (12,9 mmol) des Produktes aus Präparation 13 und 2,0 ml (12,9 mmol) Methansulfonsäurechlorid wurden analog Beispiel 4 umgesetzt. Man erhielt 1,5 g 1-(4-Methansulfonamidophenyl)-4-(N,N-(4-methansulfonamido-benzyl)methylamino)piperidin. Schmp. 177-179°C.

¹H-NMR (D₆-DMSO): δ = 1.6 (2H), 1.8 (2H), 2.1 (3H), 2.5-2.7 (3H), 2.8 (3H), 2.9 (3H), 3.5 (2H), 3.7 (2H), 6.9 (2H), 7.1 (2H), 7.15 (2H), 7.25 (2H), 9.2 (1H) und 9.65 (1H) ppm.

### Beispiel 38

3,0 g (13,6 mmol) 1-(4-Nitrophenyl)-4-piperidon und 2,0 g (13,6 mmol) N,N-(4-Methoxybenzyl)methylamin wurden analog Beispiel 1 umgesetzt. Man erhielt 3,5 g des 4-(N,N-(4-Methoxybenzyl)methylamino)-1-(4-nitrophenyl)piperidins. Schmp. 144-145°C.

### Beispiel 39

1,0 g (3.2 mmol) des Amins aus Präparation 14 wurde analog Beispiel 30 diazotiert und in Gegenwart von 0,28 g Natriumchlorid verkocht. Man erhielt 0,8 g des 1-(4-Chlorphenyl)-4-(N,N-(4-fluorbenzyl)methylamino)piperidins. Schmp. 72-73°C.

### Beispiel 40

2,4 g (10,9 mmol) 1-(4-Nitrophenyl)-4-piperidon und 2,0 g (10,9 mmol) N,N-Methyl(2-(4-nitrophenyl)-1-ethyl)amin wurden analog Beispiel 1 umgesetzt. Man erhielt 2,2 g des 1-(4-Nitrophenyl)-4-(N,N-methyl(2-(4-nitrophenyl)-1-ethyl)-amino)piperidins. Schmp. 97-98°C.

### Antiarrhythmische Wirkung

Die Wirkung der Phenylpiperidinoylamine I als Repolarisationshemmstoffe kann man durch EKG-Messungen belegen. Dabei unterteilt man die Herzperiode zeitlich in Systole (Zusammenziehen des Herzens), auch QT-Dauer genannt, und Diastole (Erschlaffung des Herzens mit Blutfüllung der Herzkammern). Repolarisationshemmstoffe bewirken nun eine Verlängerung der QT-Dauer, ohne die Vorhofkammer-Überleitungszeit (PQ-Dauer) und die Anspannungszeit (QRS-Dauer, Beginn der Systole bis zum Öffnen der Semilunarklappen) wesentlich zu verändern.

Die Wirksamkeit der erfindungsgemäßen Verbindungen als Repolarisationshemmstoffe läßt sich im Tierversuch durch EKG-Messungen beispielsweise am Meerschweinchenherzen untersuchen (s. Basic Res. Cardiol. 82 (1987) 437; J. Pharmacol. Methods 21 (1989) 195). Zum Vergleich der Wirksamkeit mehrerer Substanzen dient dabei zum Beispiel die Dosis eines Wirkstoffes, bei der eine 20 %ige Zunahme des QT-Ausgangswertes eintritt (ED_{20 %}). Hierzu trägt man die logarithmierten Dosiswerte der jeweiligen Substanzen gegen die experimentell gefundenen relativen Änderungen der QT-Dauer auf, und ermittelt mittels linearer Regression die Funktionsgleichung einer Geraden, aus der man dann den ED_{20 %}-Wert berechnen kann.

Als Versuchstiere dienten männliche Duncin-Hartley-Meerschweinchen mit einem Körpergewicht von 300 bis 350 g. 30 min nach Applikation von 1250 I.E. Heparin/kg Körpergewicht in den Bauchraum wurden die Tiere durch Genickschlag getötet. Nach dem Durchtrennen der Kopfschlagadern zum Entbluten wurde der Bruststamm geöffnet, das Herz herausgetrennt und an eine Perfusionsapparatur angeschlossen. Die Perfusion erfolgte nach Langendorff mit an Sauerstoff angereicherter, auf 37°C erwärmter Krebs-Henseleit-Lösung (NaCl 6896 mg/l; KCl 350 mg/l; MgSO₄ 285 mg/l; CaCl₂ 370 mg/l; KH₂PO₄ 161 mg/l; NaHCO₃ 2090 mg/l; Glukose 2000 mg/l). Dabei wurde das Perfusionsvolumen pro Zeiteinheit bei einem Gesamtvolumen von 100 ml auf 4 bis 6 ml/min und der Perfusionsdruck auf 60 bis 70 mm Hg eingestellt. Nach einer Äquilibrierzeit von 30 min wurde zirkulierend perfundiert.

Die EKG-Messungen wurden über zwei Silberelektroden, die auf der Herzoberfläche im oberen Bereich der linken Koronararterie und auf der Rückseite des Herzens auf Höhe der Ventilebene angebracht waren, aufgenommen. Gemessen wurden die PQ-, QT- und QRS-Zeiten sowie die Herzfrequenz.

Die Substanzgabe erfolgte kumulativ im Abstand von 15 min ins Perfusat.

### Bindung an Sigma-Rezeptor

Der verwendete Bindungsassay (Bindung von [³H]-Ditolylguanidin) erfaßt sogenannte "Haloperidolsensitive-Sigma-Rezeptoren", die eine hohe Affinität zum Haloperidol, aber nur geringe Affinität zum Phencyclidin und zu Opoiden aufweisen.

### Methoden:

### α) Membranpreparation

Rattengroßhirne wurden in dem 10fachen Volumen Homogenisierungspuffer (50 mmol Tris(hydroxymethyl)-amino-methan, 0,1 mmol Ethylendiamintetraacetat, pH=7,7) mit einem Polytron-Homogenisator homogenisiert (20 sec.). Nach 15 Minuten Zentrifugation bei 40000 Umdrehungen/min wurde das erhaltene Pellet resuspendiert und die Suspension erneut 15 Minuten lang mit 40000 Umdrehungen/min zentrifugiert. Das hierbei erhaltene Pellet wurde im 5fachen Volumen Homogenisierungspuffer resuspendiert und bis zur weiteren Verwendung in flüssigem Stickstoff eingefroren.

### β) Sigma-Bindungstest

Testsubstanz und Membranen (0,3 mg Protein) wurden in 0,3 ml Inkubationspuffer (5 mmol Tris(hydroxymethyl)-amino-methan, 0,1 mmol Ethylendiamintetraacetat, pH=7,7) 45 Minuten bei 37°C inkubiert. Nach Zugabe von 100 000 dpm [³H]-Ditolylguanidin (54,5 Ci/mmol) wurde noch 1 Stunde inkubiert. Die Membranen wurden über GF/B-Filter (dunn-Labortechnik, Asbach) filtriert und mit einem 37°C warmen Waschpuffer (5 mmol Tris(hydroxymethyl)-amino-methan, 0,1 mmol Ethylendiamintetraacetat, pH=7,4) gewaschen. Die verbleibende Radioaktivität auf den Filtern wurde durch Flüssig-Scintillationszählung gemessen. Die Bindungsdaten wurden durch iterative Anpassungsprogramme analysiert.

### ATP-abhängiger K⁺-Strom

An isolierten ventrikulären Myozyten des Meerschweinchens wurde der ATP-abhängige K⁺-Strom (A. Noma, Nature 305, 147-148 (1983)) mit der Patch-Clamp-Technik in der whole-cell-Konfiguration (O.P. Hamill et al., Plügers Arch. 391, 85-100 (1981)) gemessen. Mit einer Spannungsrampe von -100 mV bis 60 mV wurde die I/V-Kurve des gesamten K⁺-Stromes der Ventrikelzellen aufgenommen. Der ATP-abhängige K⁺-Strom wurde entweder mit Dinitrophenol (W.J. Lederer et al., J. Physiol. (London) 413, 329-349 (1989)) oder mit Cromakalim (D. Escande et al., Biochem. Biophys. Res. Comm. 154, 620-625 (1988)) aktiviert. Ein selektiver Blocker für den ATP-abhängigen K⁺-Strom in Ventrikelzellen und β-Zellen ist der Sulfonylharnstoff Glibenclamid (S.J.H. Ashcroft et al., Cellular Signalling 2, 197-214 (1990)).

## Patentansprüche

1. Phenylpiperidinoylamine der Formel I 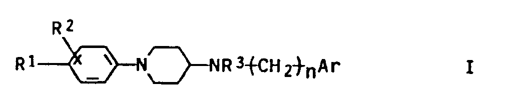 worin
R¹ H, NO₂, R⁴SO₂NH, 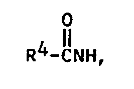 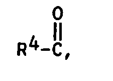 N≡C, CF₃, CF₃O, F, Cl, Br, C₁-C₄-Alkyl, R³O, CO₂R³, CHO, CH=NOR³, CH₃OR³ und
R² H, F, Cl, Br, C₁-C₄-Alkyl und R⁴O bedeuten, wobei R¹ und R² nicht gleichzeitig H sein können,
R³ H und R⁴,
R⁴ C₁-C₄-Alkyl und Phenyl,
n 1, 2, 3 und 4 und
Ar 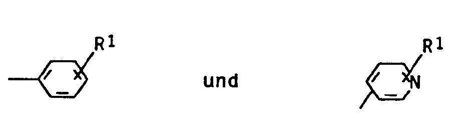 bedeutet, wobei R¹ unabhängig von R¹ in Formel I eine der dort angegebenen Bedeutungen hat,
sowie deren physiologisch verträgliche Salze.

2. Arzneimittel, das neben üblichen Hilfsstoffen pro Dosis als Wirkstoff 50 bis 1750 mg einer Verbindung nach Anspruch 1 enthält.

## Claims

1. A phenylpiperidinoylamine of the formula I 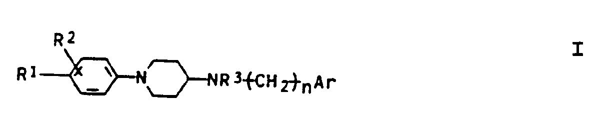 where
R¹ is H, NO₂, R⁴SO₂NH, 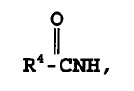 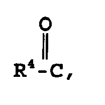 N≡C, CF₃, CF₃O, F, Cl, Br, C₁-C₄-alkyl, R³O, CO₂R³, CHO, CH=NOR³, CH₃OR³ and
R² is H, F, Cl, Br, C₁-C₄-alkyl or R⁴O, where R¹ and R² are not both H,
R³ is H or R⁴,
R⁴ is C₁-C₄-alkyl or phenyl,
n is 1, 2, 3 or 4 and
Ar is 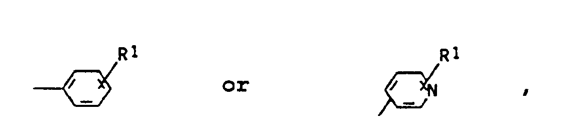 where R¹ independently of R¹ in formula I has one of the meanings stated there,
and the physiologically tolerated salts thereof.

2. A drug which contains from 50 to 1,750 mg of a compound as claimed in claim 1 per dose as active ingredient in addition to conventional ancillary substances.

## Revendications

1. Phénylpiperidinoylamines de la formule I 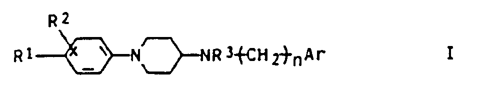 où
R¹ représente H, NO₂, R⁴SO₂NH, 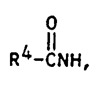 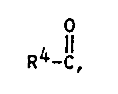 N≡C, CF₃, CF₃O, F, Cl, Br, C₁-C₄-Alkyl, R³O, CO₂R³, CHO, CH=NOR³, CH₃OR³
R² H, F, C1, Br, alkyle en C₁-C₄ et R⁴O, R¹ et R² ne pouvant pas être simultanément H
R3 représente H et R⁴,
R4 alkyle en C1-C4 et phényle,
n 1, 2, 3 et 4 et
Ar représente 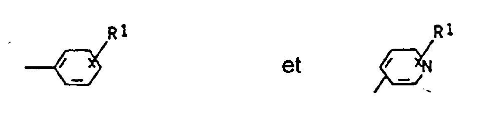 R¹ indépendant de R¹ dans la formule I a une des significations qui y est indiquée
ainsi que leurs sels acceptables physiologiquement.

2. Médicament qui, à côté d'auxiliaires usuels, contient, par dose, comme principe actif, 50 à 1750 mg d'un composé selon la revendication 1.
